# EUROPEAN PATENT SPECIFICATION

(11) **EP 1 654 542 B1**
(45) Date of publication and mention of the grant of the patent: **24.12.2008**
(21) Application number: 04763899.4
(22) Date of filing: 06.08.2004
(51) Int. Cl.: G01N 33/574

(54) **USE OF PROTEIN SPERMIDINE SYNTHASE (SPEE) AS A MARKER FOR COLORECTAL CANCER**
VERWENDUNG DES PROTEINS SPERMIDINSYNTHASE (SPEE) ALS MARKER FÜR KOLOREKTALKARZINOM
UTILISATION DE LA PROTEINE SPERMIDINE SYNTHASE (SPEE) COMME MARQUEUR DU CANCER COLORECTAL

(30) Priority: 08.08.2003 EP 03017585
(43) Date of publication of application: 10.05.2006
(73) Proprietor: Roche Diagnostics GmbH, 68305 Mannheim (DE); F. Hoffmann-La Roche AG, 4070 Basel (CH)
(72) Inventor: TACKE, Michael, 81373 Muenchen (DE); BERNDT, Peter, CH-Basel (CH); HAGMANN, Marie-Luise, 82377 Penzberg (DE); KARL, Johann, 82380 Peissenberg (DE); LANGEN, Hanno, 79540 Loerrach (DE); PALME, Stefan, 82377 Penzberg (DE); ROESSLER, Markus, 82110 Germering (DE); ROLLINGER, Wolfgang, 82398 Polling (DE); ZOLG, Werner, 82362 Weilheim (DE)
(86) International application number: PCT/EP2004/008871
(87) International publication number: WO 2005/015231

(56) References cited:
- WO-A2-02/059377
- LOESER C ET AL: "POLYAMINES IN COLORECTAL CANCER EVALUATION OF POLYAMINE CONCENTRATIONS IN THE COLON TISSUE SERUM AND URINE OF 50 PATIENTS WITH COLORECTAL CANCER" CANCER, vol. 65, no. 4, 1990, pages 958-966, XP008036405 ISSN: 0008-543X
- LUK G D ET AL: "S ADENOSYL-3-THIO-1 8-DIAMINOOCTANE ADODATO SUPPRESSES THE GROWTH OF HUMAN COLON CANCER CELLS" GASTROENTEROLOGY, vol. 94, no. 5 PART 2, 1988, page A272, XP008036437 & 89TH ANNUAL MEETING OF THE AMERICAN GASTROENTEROLOGICAL ASSOCIATION, NEW ORLEANS, LOUISIANA, USA, MA ISSN: 0016-5085
- DEZEURE F.; SARHAN S.; SEILER N.: 'CHAIN-FLUORINATED POLYAMINES AS TUMOR MARKERS IV. COMPARISON OF 2 FLUOROPUTRESCINE AND 2 2 DIFLUOROPUTRESCINE AS SUBSTRATES OF SPERMIDINE SYNTHASE IN-VITRO AND IN-VIVO' INTERNATIONAL JOURNAL OF BIOCHEMISTRY vol. 20, no. 11, 1988, pages 1299 - 1312
- WEISS THOMAS S. ET AL: 'Polyamine levels of human colorectal adenocarcinomas are correlated with tumor stage and grade.' INTERNATIONAL JOURNAL OF COLORECTAL DISEASE vol. 17, no. 6, 01 November 2002, pages 381 - 387

## Description

The present invention relates to the diagnosis of colorectal cancer. It discloses the use of the protein spermidine synthase (= SPEE) in the diagnosis of colorectal cancer. Furthermore, it especially relates to a method for diagnosis of colorectal cancer from a liquid sample, derived from an individual by measuring SPEE in said sample. Measurement of SPEE can, e.g., be used in the early detection or diagnosis of colorectal cancer.

Cancer remains a major public health challenge despite progress in detection and therapy. Amongst the various types of cancer, colorectal cancer (= CRC) is one of the most frequent cancers in the Western world.

The earlier cancer can be detected/diagnosed, the better is the overall survival rate. This is especially true for CRC. The prognosis in advanced stages of tumor is poor. More than one third of the patients will die from progressive disease within five years after diagnosis, corresponding to a survival rate of about 40% for five years. Current treatment is only curing a fraction of the patients and clearly has the best effect on those patients diagnosed in an early stage of disease.

With regard to CRC as a public health problem, it is essential that more effective screening and preventative measures for colorectal cancer be developed.

The earliest detection procedures available at present for colorectal cancer involve using tests for fecal blood or endoscopic procedures. However, significant tumor size must typically exist before fecal blood is detected. The sensitivity of the guaiac-based fecal occult blood tests is ~26%, which means 74% of patients with malignant lesions will remain undetected (Ahlquist, D.A., Gastroenterol. Clin. North Am. 26 (1997) 41-55). The visualization of precancerous and cancerous lesions represents the best approach to early detection, but colonoscopy is invasive with significant costs, risks, and complications (Silvis, S.E., et al., JAMA 235 (1976) 928-930; Geenen, J.E., et al., Am. J. Dig. Dis. 20 (1975) 231-235; Anderson, W.F., et al., J. Natl. Cancer Institute 94 (2002) 1126-1133).

In the recent years a tremendous amount of so-called colon specific or even so-called colorectal cancer specific genes has been reported. The vast majority of the corresponding research papers or patent applications are based on data obtained by analysis of RNA expression patterns in colon (cancer) tissue versus a different tissue or an adjacent normal tissue, respectively. Such approaches may be summarized as differential mRNA display techniques.

As an example for data available from mRNA-display techniques, WO 01/96390 shall be mentioned and discussed. This application describes and claims more than two hundred isolated polynucleotides and the corresponding polypeptides as such, as well as their use in the detection of CRC. However, it is general knowledge that differences on the level of mRNA are not mirrored by the level of the corresponding proteins. A protein encoded by a rare mRNA may be found in very high amounts and a protein encoded by an abundant mRNA may nonetheless be hard to detect and find at all. This lack of correlation between mRNA-level and protein level is due to reasons like mRNA stability, efficiency of translation, stability of the protein, etc.

There also are recent approaches investigating the differences in protein patterns between different tissues or between healthy and diseased tissue in order to identify candidate marker molecules which might be used in the diagnosis of CRC. Brünagel, G., et al., Cancer Research 62 (2002) 2437-2442 have identified seven nuclear matrix proteins which appear to be more abundant in CRC tissue as compared to adjacent normal tissue. No data from liquid samples obtained from an individual are reported.

WO 02/078636 reports about nine colorectal cancer-associated spots as found by surface-enhanced laser desorption and ionization (SELDI). These spots are seen more frequently in sera obtained from patients with CRC as compared to sera obtained from healthy controls. However, the identity of the molecule(s) comprised in such spot, e.g., its (their sequence), is not known.

Despite the large and ever growing list of candidate protein markers in the field of CRC, to date clinical/diagnostic utility of these molecules is not known. In order to be of clinical utility a new diagnostic marker as a single marker should be at least as good as the best single marker known in the art. Or, a new marker should lead to a progress in diagnostic sensitivity and/or specificity either if used alone or in combination with one or more other markers, respectively. The diagnostic sensitivity and/or specificity of a test is best assessed by its receiver-operating characteristics, which will be described in detail below.

At present, only diagnostic blood tests based on the detection of carcinoembryonic antigen (CEA), a tumor-associated glycoprotein, are available to assist diagnosis in the field of CRC. CEA is increased in 95% of tissue samples obtained from patients with colorectal, gastric, and pancreatic cancers and in the majority of breast, lung, and head and neck carcinomas (Goldenberg, D.M., et al., J. Natl. Cancer Inst. (Bethesda) 57 (1976) 11-22). Elevated CEA levels have also been reported in patients with nonmalignant disease, and many patients with colorectal cancer have normal CEA levels in the serum, especially during the early stage of the disease (Carriquiry, L.A., and Pineyro, A., Dis. Colon Rectum 42 (1999) 921-929; Herrera, M.A., et al., Ann. Surg. 183 (1976) 5-9; Wanebo, H.J., et al., N. Engl. J. Med. 299 (1978) 448-451). The utility of CEA as measured from serum or plasma in detecting recurrences is reportedly controversial and has yet to be widely applied (Martell, R.E., et al., Int. J. Biol. Markers 13 (1998) 145-149; Moertel, C.G., et al., JAMA 270 (1993) 943-947).

In light of the available data, serum CEA determination possesses neither the sensitivity nor the specificity to enable its use as a screening test for colorectal cancer in the asymptomatic population (Reynoso, G., et al., JAMA 220 (1972) 361-365; Sturgeon, C., Clinical Chemistry 48 (2002) 1151-1159).

Whole blood, serum or plasma are the most widely used sources of sample in clinical routine. The identification of an early CRC tumor marker that would allow reliable cancer detection or provide early prognostic information could lead to a diagnostic assay that would greatly aid in the diagnosis and in the management of this disease. Therefore, an urgent clinical need exists to improve the diagnosis of CRC from blood. It is especially important to improve the early diagnosis of CRC, since for patients diagnosed early on chances of survival are much higher as compared to those diagnosed at a progressed stage of disease.

It was the task of the present invention to investigate whether a new marker can be identified which may aid in CRC diagnosis.

Surprisingly, it has been found that use of protein SPEE can at least partially overcome the problems known from the state of the art.

The present invention therefore relates to a method for the diagnosis of colorectal cancer comprising the steps of a) providing a liquid sample obtained from an individual, b) contacting said sample with a specific binding agent for SPEE under conditions appropriate for formation of a complex between said binding agent and SPEE, and c) correlating the amount of complex formed in (b) to the diagnosis of colorectal cancer.

Another preferred embodiment of the invention is a method for the diagnosis of colorectal cancer comprising the steps of a) contacting a liquid sample obtained from an individual with a specific binding agent for SPEE under conditions appropriate for formation of a complex between said binding agent and SPEE, and b) correlating the amount of complex formed in (a) to the diagnosis of colorectal cancer.

As the skilled artisan will appreciate, any such diagnosis is made in vitro. The patient sample is discarded afterwards. The patient sample is solely used for the in vitro diagnostic method of the invention and the material of the patient sample is not transferred back into the patient's body. Typically, the sample is a liquid sample.

The protein SPEE (spermidine synthase; SWISS-PROT: P19623) is characterized by the sequence given SEQ ID NO: 1. The corresponding cloned human cDNA encodes for a 34-kDa protein which is a member of spermidine/spermine synthase family. The primary structure of the cDNA as well as the chromosomal localization of the gene were resolved in the early 1990s (Wahlfors, J., et al., DNA Cell Biol. 9 (1990) 103-110; Wahlfors, J., et al., DNA Cell Biol. 9 (1990) 543; Myohanen, S., et al., DNA Cell Biol. 10 (1991) 467-474; Wingqvist, et al., Cytogenet. Cell. Genet. 58 (1991) 1865). SPEE is one of the four proteins which are involved in the biosynthesis of polyamines and catalyzes the reaction from S-adenosylmethioninamine + putrescine to 5'-methylthioadenosine + spermidine, the last step in the biosynthesis of spermidine.

Polyamines (e. g. putrescine, spermine, spermidine) are universally essential for several cellular functions (Tabor, C.W., and Tabor, H., Annu. Rev. Biochem. 53 (1984) 749-790; Janne, J., et al., Ann. Med. 23 (1991) 241-259). Early reports already described that enzyme activities of SPEE and the other enzymes involved in the biosynthesis of polyamines were increased during cell proliferation (Hannonen, P., et al., Biochim. Biophys. Acta 273 (1972) 84-90; Kapyaho, K., et al., Biochem. J. 192 (1980) 59-63; Korpela, H., et al., Biochem. J. 196 (1981) 733-738) and it is a long known fact that levels of intracellular polyamines are increased in malignant tissue as compared to healthy controls (Scalabrino, G., and Ferioli, M.E., Adv, Cancer Res. 35 (1981) 151-268). Most recent work revealed that intracellular polyamine levels in human colorectal adenocarcinoma are correlated with the tumor stage (Weiss, T.S., et al., Int. J. Colorectal Dis. 17 (2002) 381-387) and have a prognostic value (Linsalata, M., et al., Anticancer Res. 22 (2002) 2465-2469).

Consequently, there are a number of inhibitors of SPEE activity, such as dicyclohexylammonium sulfate (Ito, H., et al., Cancer Lett. 15 (1982) 229-235; Feuerstein, B.G., et al., Cancer Res. 45 (1985) 4950-4954), methyl-thiopropylamine (Hibasami, H., et al., Anticancer Res. 7 (1987) 1213-1216) and mercaptoetylamine (Hibasami, H., et al., FEBS Lett. 229 (1988) 243-246) which are reported to have a growth retarding effect on tumor cells.

Nishikawa, et al. performed transcription analyses to show that expression of the gene encoding SPEE is inhibited in hepatoma cells by transforming growth factor-β₁ (TGF-β₁) (Nishikawa, Y., et al., Biochem. J. 321 (1997) 537-543).

Luk, G.D., et al Gastroenterology 94/5 (1988) A272, have used six human colon carcinoma cell lines and have investigated polyamines in comparison to the differentiation status of the various cell lines. Loeser, C., et al., Cancer 65 (1990) 958-966, have investigated polyamines in colon cancer and evaluated the level of various polyamines in colon tissue, serum and urine, respectively.

As obvious to the skilled artisan, the present invention shall not be construed to be limited to the full-length protein SPEE of SEQ ID NO: 1. Physiological or artificial fragments of SPEE, secondary modifications of SPEE, as well as allelic variants of

SPEE are also encompassed by the present invention. Artificial fragments preferably encompass a peptide produced synthetically or by recombinant techniques, which at least comprises one epitope of diagnostic interest consisting of at least 6 contiguous amino acids as derived from the sequence disclosed in SEQ ID NO:1. Such fragment may advantageously be used for generation of antibodies or as a standard in an immunoassay. More preferred the artificial fragment comprises at least two epitopes of interest appropriate for setting up a sandwich immunoassay.

In preferred embodiments, the novel marker SPEE may be used for monitoring as well as for screening purposes.

When used in patient monitoring the diagnostic method according to the present invention may help to assess tumor load, efficacy of treatment and tumor recurrence in the follow-up of patients. Increased levels of SPEE are directly correlated to tumor burden. After chemotherapy a short term (few hours to 14 days) increase in SPEE may serve as an indicator of tumor cell death. In the follow-up of patients (from 3 months to 10 years) an increase of SPEE can be used as an indicator for tumor recurrence.

In a preferred embodiment the diagnostic method according to the present invention is used for screening purposes. I.e., it is used to assess subjects without a prior diagnosis of CRC by measuring the level of SPEE and correlating the level measured to the presence or absence of CRC.

Colorectal cancer most frequently progresses from adenomas (polyps) to malignant carcinomas. The different stages of CRC used to be classified according to Dukes' stages A to D.

The staging of cancer is the classification of the disease in terms of extent, progression, and severity. It groups cancer patients so that generalizations can be made about prognosis and the choice of therapy.

Today, the TNM system is the most widely used classification of the anatomical extent of cancer. It represents an internationally accepted, uniform staging system. There are three basic variables: T (the extent of the primary tumor), N (the status of regional lymph nodes) and M (the presence or absence of distant metastases). The TNM criteria are published by the UICC (International Union Against Cancer), Sobin, L.H., Wittekind, Ch. (eds): TNM Classification of Malignant Tumours, fifth edition, 1997.

What is especially important is, that early diagnosis of CRC translates to a much better prognosis. Malignant tumors of the colorectum arise from benign tumors, i.e. from adenoma. Therefore, best prognosis have those patients diagnosed at the adenoma stage. Patients diagnosed as early as in stage Tᵢₛ, N0, M0 or T1-3; N0; M0, if treated properly have a more than 90% chance of survival 5 years after diagnosis as compared to a 5-years survival rate of only 10% for patients diagnosed when distant metastases are already present.

In the sense of the present invention early diagnosis of CRC refers to a diagnosis at a pre-malignant state (adenoma) or at a tumor stage where no metastases at all (neither proximal nor distal), i.e., adenoma, Tᵢₛ, N0, M0 or T1-4; N0; M0 are present. Tᵢₛ denotes carcinoma *in situ*.

In a preferred embodiment SPEE is used to diagnose CRC as early as in the adenoma stage.

It is further preferred, that CRC is diagnosed when it has not yet fully grown through the bowel wall and thus neither the visceral peritoneum is perforated nor other organs or structures are invaded, i.e., that diagnosis is made at stage Tᵢₛ, N0, M0 or T1-3; N0; M0 (=Tᵢₛ-3; N0; M0).

The diagnostic method according to the present invention is based on a liquid sample which is derived from an individual. Unlike to methods known from the art SPEE is specifically measured from this liquid sample by use of a specific binding agent.

A specific binding agent is, e.g., a receptor for SPEE, a lectin binding to SPEE or an antibody to SPEE. A specific binding agent has at least an affinity of 10⁷l/mol for its corresponding target molecule. The specific binding agent preferably has an affinity of 10⁸ l/mol or even more preferred of 10⁹ l/mol for its target molecule. As the skilled artisan will appreciate the term specific is used to indicate that other biomolecules present in the sample do not significantly bind to with the binding agent specific for SPEE. Preferably, the level of binding to a biomolecule other than the target molecule results in a binding affinity which is only 10%, more preferably only 5% of the affinity of the target molecule or less. A most preferred specific binding agent will fulfill both the above minimum criteria for affinity as well as for specificity.

A specific binding agent preferably is an antibody reactive with SPEE. The term antibody refers to a polyclonal antibody, a monoclonal antibody, fragments of such antibodies, as well as to genetic constructs comprising the binding domain of an antibody.

Any antibody fragment retaining the above criteria of a specific binding agent can be used. Antibodies are generated by state of the art procedures, e.g., as described in Tijssen (Tijssen, P., Practice and theory of enzyme immunoassays 11 (1990) the whole book, especially pages 43-78; Elsevier, Amsterdam). In addition, the skilled artisan is well aware of methods based on immunosorbents that can be used for the specific isolation of antibodies. By these means the quality of polyclonal antibodies and hence their performance in immunoassays can be enhanced. (Tijssen, P., *supra,* pages 108-115).

For the achievements as disclosed in the present invention polyclonal antibodies raised in rabbits have been used. However, clearly also polyclonal antibodies from different species , e.g. rats or guinea pigs, as well as monoclonal antibodies can also be used. Since monoclonal antibodies can be produced in any amount required with constant properties, they represent ideal tools in development of an assay for clinical routine. The generation and use of monoclonal antibodies to SPEE in a method according to the present invention is yet another preferred embodiment.

As the skilled artisan will appreciate now, that SPEE has been identified as a marker which is useful in the diagnosis of CRC, alternative ways may be used to reach a result comparable to the achievements of the present invention. For example, alternative strategies to generate antibodies may be used. Such strategies comprise amongst others the use of synthetic peptides, representing an epitope of SPEE for immunization. Alternatively, DNA Immunization also known as DNA vaccination may be used.

For measurement the liquid sample obtained from an individual is incubated with the specific binding agent for SPEE under conditions appropriate for formation of a binding agent SPEE-complex. Such conditions need not be specified, since the skilled artisan without any inventive effort can easily identify such appropriate incubation conditions.

As a final step according to the method disclosed in the present invention the amount of complex is measured and correlated to the diagnosis of CRC. As the skilled artisan will appreciate there are numerous methods to measure the amount of the specific binding agent SPEE-complex all described in detail in relevant textbooks (cf., e.g., Tijssen P., *supra,* or Diamandis, et al., eds. (1996) Immunoassay, Academic Press, Boston).

Preferably SPEE is detected in a sandwich type assay format. In such assay a first specific binding agent is used to capture SPEE on the one side and a second specific binding agent, which is labeled to be directly or indirectly detectable is used on the other side.

As mentioned above, it has surprisingly been found that SPEE can be measured from a liquid sample obtained from an individual sample. No tissue and no biopsy sample is required to apply the marker SPEE in the diagnosis of CRC.

In a preferred embodiment the method according to the present invention is practiced with serum as liquid sample material.

In a further preferred embodiment the method according to the present invention is practiced with plasma as liquid sample material.

In a further preferred embodiment the method according to the present invention is practiced with whole blood as liquid sample material.

Furthermore stool can be prepared in various ways known to the skilled artisan to result in a liquid sample as well. Such sample liquid derived from stool also represents a preferred embodiment according to the present invention.

Whereas application of routine proteomics methods to tissue samples, leads to the identification of many potential marker candidates for the tissue selected, the inventors of the present invention have surprisingly been able to detect protein SPEE in a bodily fluid sample. Even more surprising they have been able to demonstrate that the presence of SPEE in such liquid sample obtained from an individual can be correlated to the diagnosis of colorectal cancer.

Antibodies to SPEE with great advantage can be used in established procedures, e.g., to detect colorectal cancer cells in situ, in biopsies, or in immunohistological procedures.

Preferably, an antibody to SPEE is used in a qualitative (SPEE present or absent) or quantitative (SPEE amount is determined) immunoassay.

Measuring the level of protein SPEE has proven very advantageous in the field of CRC. Therefore, in a further preferred embodiment, the present invention relates to use of protein SPEE as a marker molecule in the diagnosis of colorectal cancer from a liquid sample obtained from an individual.

The term marker molecule is used to indicate that an increased level of the analyte SPEE as measured from a bodily fluid of an individual marks the presence of CRC.

It is especially preferred to use the novel marker SPEE in the early diagnosis of colorectal cancer.

The use of protein SPEE itself, represents a significant progress to the challenging field of CRC diagnosis. Combining measurements of SPEE with other known markers, like CEA, or with other markers of CRC yet to be discovered, leads to further improvements. Therefore in a further preferred embodiment the present invention relates to the use of SPEE as a marker molecule for colorectal cancer in combination with one or more marker molecules for colorectal cancer in the diagnosis of colorectal cancer from a liquid sample obtained from an individual. In this regard, the expression "one or more" denotes 1 to 10, preferably 1 to 5, more preferred 3. Preferred selected other CRC markers with which the measurement of SPEE may be combined are CEA, CA 19-9, CA 72-4, and/or CA 242. Thus, a very much preferred embodiment of the present invention is the use of protein SPEE as a marker molecule for colorectal cancer in combination with one or more marker molecules for colorectal cancer in the diagnosis of colorectal cancer from a liquid sample obtained from an individual, whereby the at least one other marker molecule is selected from the group consisting of CEA, CA 19-9, CA 72-4, and CA 242. Very preferred the marker SPEE is used in combination with CEA.

Accuracy of a test is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results) (number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results) / (number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Clinical utility of the novel marker SPEE has been assessed in comparison to and in combination with the established marker CEA, using a receiver operator curve analysis (ROC; Zweig, M. H., and Campbell, G., Clin. Chem. 39 (1993) 561-577). This analysis has been based on well-defined patient cohorts consisting of 50 samples each from patients in T1-3; N0; M0, more progressed tumor, i.e., T4 and/or various severity of metastasis (N+ and/or M+), and healthy controls, respectively.

### Description of the Figures

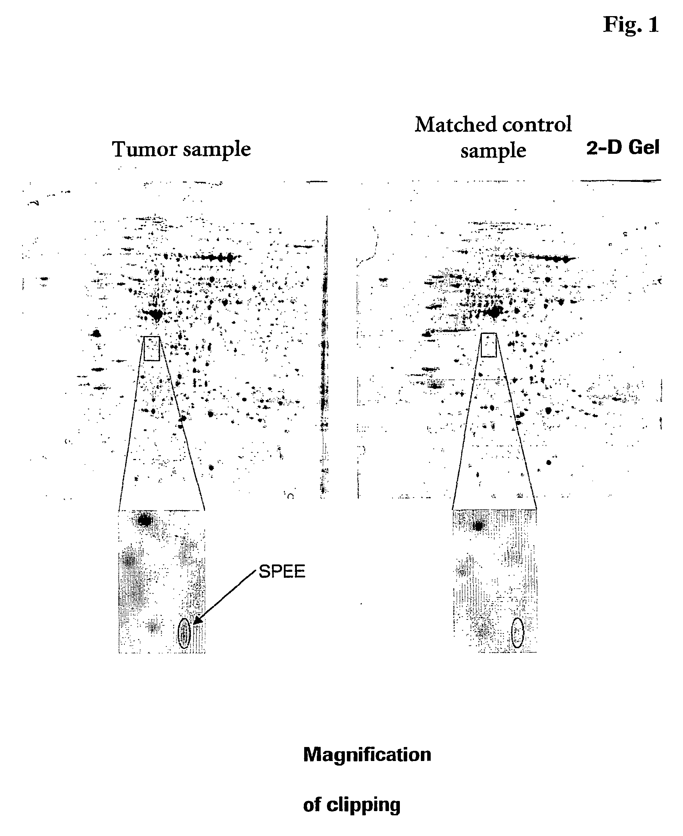

- **Figure 1**: Identification of SPEE with an apparent MW of 34 kDa and an isoelectric point at about pH 5 (higher) in colon tumor tissue. Figure 1 shows a typical example of a 2D-gel, loaded with a tumor sample (left side), and a gel, loaded with a matched control sample (right side) obtained from adjacent healthy mucosa. The circle in the enlarged section of these gels indicates the position for the protein SPEE. This protein was not detectable by the same method in healthy mucosa.

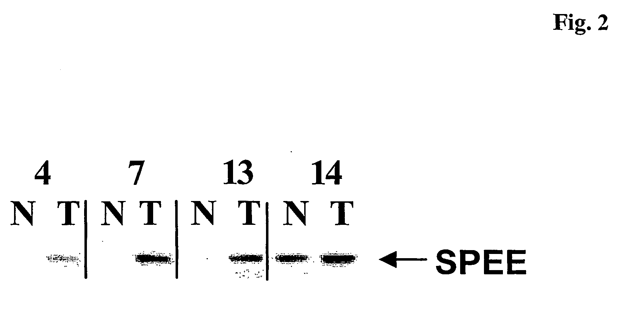

- **Figure 2**: Typical example of a Western-Blot. The polyacrylamide gel was loaded with tissue lysates from colorectal tumor tissue and adjacent healthy control tissue from 4 patients (subject 4: colon ca (carcinoma), Dukes C; subject 7: colon ca, Dukes C; subject 13: colon ca, Dukes B; and subject 14: colon ca, Dukes B) and after electrophoresis the proteins were blotted onto a nitrocellulose membrane. Presence of SPEE in the samples was tested using a polyclonal rabbit anti-SPEE serum. Lanes containing tumor lysates are indicated with "T", lanes containing normal control tissue with "N". The arrow indicates the position in the gel of the SPEE band. All tumor samples give a strong signal at the position of SPEE, whereas only a weak signal can be detected in the lysates from adjacent normal control tissue.

### Abbreviations

- ABTS: 2,2'-Azino-di- [3-ethylbenzthiazoline sulfonate (6)] diammonium salt
- BSA: bovine serum albumin
- cDNA: complementary DNA
- CHAPS: (3-[(3-Cholamidopropyl)-dimethylammonio]- 1-propane- sulfonate)
- DMSO: dimethyl sulfoxide
- DTT: dithiothreitol
- EDTA: ethylene diamine tetraacetic acid
- ELISA: enzyme-linked immunosorbent assay
- HRP: horseradish peroxidase
- IAA: iodoacetamid
- IgG: immunoglobulin G
- IEF: isoelectric focussing
- IPG: immobilized pH gradient
- LDS: lithium dodecyl sulfate
- MALDI-TOF: matrix-assisted laser desorption/ionisation-time of flight mass spectrometry
- MES: mesityl, 2,4,6-trimethylphenyl
- OD: optical density
- PAGE: polyacrylamide gel electrophoresis
- PBS: phosphate buffered saline
- PI: isoelectric point
- RTS: rapid translation system
- SDS: sodium dodecyl sulfate

### Example 1

### Identification of SPEE as a potential colorectal cancer marker

### Sources of tissue

In order to identify tumor-specific proteins as potential diagnostic markers for colorectal cancer, analysis of three different kinds of tissue using proteomics methods is performed.

In total, tissue specimen from 10 patients suffering from colorectal cancer are analyzed. From each patient three different tissue types are collected from therapeutic resections: tumor tissue (> 80% tumor) (T), adjacent healthy tissue (N) and stripped mucosa from adjacent healthy mucosa (M). The latter two tissue types serve as matched healthy control samples. Tissues are immediately snap frozen after resection and stored at - 80°C before processing. Tumors are diagnosed by histopathological criteria.

### Tissue preparation

0.8-1.2 g of frozen tissue are put into a mortar and completely frozen by liquid nitrogen. The tissue is pulverized in the mortar, dissolved in the 10-fold volume (w/v) of lysis buffer (40 mM Na-citrate, 5 mM MgCl₂, 1% Genapol X-080, 0.02% Na-azide, Complete^{®} EDTA-free [Roche Diagnostics GmbH, Mannheim, Germany, Cat. No. 1 873 580]) and subsequently homogenized in a Wheaton^{®} glass homogenizer (20 x loose fitting, 20 x tight fitting). 3 ml of the homogenate are subjected to a sucrose-density centrifugation (10-60% sucrose) for 1 h at 4500 x g. After this centrifugation step three fractions are obtained. The fraction on top of the gradient contains the soluble proteins and is used for further analysis.

### Isoelectric focussing (IEF) and SDS-PAGE

For IEF, 3 ml of the suspension are mixed with 12 ml sample buffer (7 M urea, 2 M thiourea, 2% CHAPS, 0.4% IPG buffer pH 4-7, 0.5% DTT) and incubated for 1 h. The samples are concentrated in an Amicon^{®} Ultra-15 device (Millipore GmbH, Schwalbach, Germany) and the protein concentration is determined using the Bio-Rad^{®} protein assay (Cat.No. 500-0006; Bio-Rad Laboratories GmbH, München, Germany) following the instructions of the supplier's manual. To a volume corresponding to 1.5 mg of protein sample buffer is added to a final volume of 350 µl. This solution is used to rehydrate IPG strips pH 4-7 (Amersham Biosciences, Freiburg, Germany) overnight. The IEF is performed using the following gradient protocol: 1.) 1 minute to 500 V; 2.) 2 h to 3,500 V; 3.) 22 h at constant 3,500 V giving rise to 82 kVh. After IEF, strips are stored at -80°C or directly used for SDS-PAGE.

Prior to SDS-PAGE the strips are incubated in equilibration buffer (6 M urea, 50 mM Tris/HCl, pH 8.8, 30% glycerol, 2% SDS), for reduction DDT (15 min, + 50 mg DTT/10 ml), and for alkylation IAA (15 min, + 235 mg iodacetamide/10 ml) is added. The strips are put on 12.5% polyacrylamide gels and subjected to electrophoresis at 1 W/gel for 1 h and thereafter at 17 W/gel. Subsequently, the gels are fixed (50% methanol, 10% acetate) and stained overnight with Novex^{™} Colloidal Blue Staining Kit (Invitrogen, Karlsruhe, Germany, Cat No. LC6025, 45-7101).

### Detection of SPEE as a potential marker for colorectal cancer

Each patient is analyzed separately by image analysis with the ProteomeWeaver^{®} software (Definiens AG, Germany, München). In addition, all spots of the gel are excised by a picking robot and the proteins present in the spots are identified by MALDI-TOF mass spectrometry (Ultraflex^{™} Tof/Tof, Bruker Daltonik GmbH, Bremen, Germany). For each patient, 4 gels from the tumor sample are compared with 4 gels each from adjacent normal and stripped mucosa tissue and analyzed for distinctive spots corresponding to differentially expressed proteins. By this means, protein SPEE is found to be specifically expressed or strongly overexpressed in tumor tissue and not detectable or less strongly expressed in healthy control tissue. It therefore - amongst many other proteins - qualifies as a candidate marker for use in the diagnosis of colorectal cancer.

### Example 2

### Generation of antibodies to the colorectal cancer marker protein SPEE

Polyclonal antibody to the colorectal cancer marker protein SPEE is generated for further use of the antibody in the measurement of serum and plasma and blood levels of SPEE by immunodetection assays, e.g. Western Blotting and ELISA.

### Recombinant protein expression in E. coli

In order to generate antibodies to SPEE, recombinant expression of the protein is performed for obtaining immunogens. The expression is done applying a combination of the RTS 100 expression system and E.coli. In a first step, the DNA sequence is analyzed and recommendations for high yield cDNA silent mutational variants and respective PCR-primer sequences are obtained using the "ProteoExpert RTS E.coli HY" system. This is a commercial web based service (www.proteoexpert.com). Using the recommended primer pairs, the "RTS 100 E. coli Linear Template Generation Set, His-tag" (Roche Diagnostics GmbH, Mannheim, Germany, Cat.No. 3186237) system to generate linear PCR templates from the cDNA and for in-vitro transcription and expression of the nucleotide sequence coding for the SPEE protein is used. For Western-blot detection and later purification, the expressed protein contains a His-tag. The best expressing variant is identified. All steps from PCR to expression and detection are carried out according to the instructions of the manufacturer. The respective PCR product, containing all necessary T7 regulatory regions (promoter, ribosomal binding site and T7 terminator) is cloned into the pBAD TOPO^{®} vector (Invitrogen, Karlsruhe, Germany, Cat. No. K 4300/01) following the manufacturer's instructions. For expression using the T7 regulatory sequences, the construct is transformed into E. coli BL 21 (DE 3) (Studier, F.W., et al., Methods Enzymol. 185 (1990) 60-89) and the transformed bacteria are cultivated in a 11 batch for protein expression.

Purification of His-SPEE fusion protein is done following standard procedures on a Ni-chelate column. Briefly, 1l of bacteria culture containing the expression vector for the His-SPEE fusion protein is pelleted by centrifugation. The cell pellet is resuspended in lysis buffer, containing phosphate, pH 8.0, 7 M guanidium chloride, imidazole and thioglycerole, followed by homogenization using a Ultra-Turrax^{®}. Insoluble material is pelleted by high speed centrifugation and the supernatant is applied to a Ni-chelate chromatographic column. The column is washed with several bed volumes of lysis buffer followed by washes with buffer, containing phosphate, pH 8.0 and Urea. Finally, bound antigen is eluted using a phosphate buffer containing SDS under acid conditions.

### Production of monoclonal antibodies Against the SPEE

### a) Immunization of mice

12 week old A/J mice are initially immunized intraperitoneally with 100 µg SPEE. This is followed after 6 weeks by two further intraperitoneal immunizations at monthly intervals. In this process each mouse is administered 100 µg SPEE adsorbed to aluminum hydroxide and 10⁹ germs of *Bordetella pertussis*. Subsequently the last two immunizations are carried out intravenously on the 3rd and 2nd day before fusion using 100 µg SPEE in PBS buffer for each.

### b) Fusion and cloning

Spleen cells of the mice immunized according to a) are fused with myeloma cells according to Galfre, G., and Milstein, C., Methods in Enzymology 73 (1981) 3-46. In this process ca. 1*10⁸ spleen cells of the immunized mouse are mixed with 2x10⁷ myeloma cells (P3X63-Ag8-653, ATCC CRL1580) and centrifuged (10 min at 300 x g and 4°C.). The cells are then washed once with RPMI 1640 medium without fetal calf serum (FCS) and centrifuged again at 400 x g in a 50 ml conical tube. The supernatant is discarded, the cell sediment is gently loosened by tapping, 1 ml PEG (molecular weight 4000, Merck, Darmstadt) is added and mixed by pipetting. After 1 min in a water-bath at 37°C., 5 ml RPMI 1640 without FCS is added drop-wise at room temperature within a period of 4-5 min. Afterwards 5 ml RPMI 1640 containing 10% FCS is added drop-wise within ca. 1 min, mixed thoroughly, filled to 50 ml with medium (RPMI 1640+10% FCS) and subsequently centrifuged for 10 min at 400 x g and 4°C. The sedimented cells are taken up in RPMI 1640 medium containing 10% FCS and sown in hypoxanthine-azaserine selection medium (100 mmol/l hypoxanthine, 1 µg/ml azaserine in RPMI 1640+10% FCS). Interleukin 6 at 100 U/ml is added to the medium as a growth factor.

After ca. 10 days the primary cultures are tested for specific antibody. SPEE-positive primary cultures are cloned in 96-well cell culture plates by means of a fluorescence activated cell sorter. In this process again interleukin 6 at 100 U/ml is added to the medium as a growth additive.

### c) Immunoglobulin isolation from the cell culture supernatants

The hybridoma cells obtained are sown at a density of 1x10⁵ cells per ml in RPMI 1640 medium containing 10% FCS and proliferated for 7 days in a fermenter (Thermodux Co., Wertheim/Main, Model MCS-104XL, Order No. 144-050). On average concentrations of 100 µg monoclonal antibody per ml are obtained in the culture supernatant. Purification of this antibody from the culture supernatant is carried out by conventional methods in protein chemistry (e.g. according to Bruck, C., et al., Methods in Enzymology 121 (1986) 587-695).

### Generation of polyclonal antibodies

### a) Immunization

For immunization, a fresh emulsion of the protein solution (100 µg/ml protein SPEE) and complete Freund's adjuvant at the ratio of 1:1 is prepared. Each rabbit is immunized with 1 ml of the emulsion at days 1, 7, 14 and 30, 60 and 90. Blood is drawn and resulting anti-SPEE serum used for further experiments as described in examples 3 and 4.

### b) Purification of IgG (immunoglobulin G) from rabbit serum by sequential precipitation with caprylic acid and ammonium sulfate

One volume of rabbit serum is diluted with 4 volumes of acetate buffer (60 mM, pH 4.0). The pH is adjusted to 4.5 with 2 M Tris-base. Caprylic acid (25 µl/ml of diluted sample) is added drop-wise under vigorous stirring. After 30 min the sample is centrifuged (13,000 x g, 30 min, 4°C), the pellet discarded and the supernatant collected. The pH of the supernatant is adjusted to 7.5 by the addition of 2 M Tris-base and filtered (0.2 µm).

The immunoglobulin in the supernatant is precipitated under vigorous stirring by the drop-wise addition of a 4 M ammonium sulfate solution to a final concentration of 2 M. The precipitated immunoglobulins are collected by centrifugation (8,000 x g,15 min, 4°C).

The supernatant is discarded. The pellet is dissolved in 10 mM NaH₂PO₄/NaOH, pH 7.5, 30 mM NaCl and exhaustively dialyzed. The dialysate is centrifuged (13,000 x g, 15 min, 4°C) and filtered (0.2 µm).

### Biotinylation of polyclonal rabbit IgG

Polyclonal rabbit IgG is brought to 10 mg/ml in 10 mM NaH₂PO₄/NaOH, pH 7.5, 30 mM NaCl. Per ml IgG solution 50 µl Biotin -N-hydroxysuccinimide (3.6 mg/ml in DMSO) are added. After 30 min at room temperature, the sample is chromatographed on Superdex 200 (10 mM NaH₂PO₄/NaOH, pH 7.5, 30 mM NaCl). The fraction containing biotinylated IgG are collected. Monoclonal antibodies are biotinylated according to the same procedure.

### Digoxygenylation of polyclonal rabbit IgG

Polyclonal rabbit IgG is brought to 10 mg/ml in 10 mM NaH₂PO₄/NaOH, 30 mM NaCl, pH 7.5. Per ml IgG solution 50 µl digoxigenin-3-O-methylcarbonyl-ε-aminocaproic acid-N-hydroxysuccinimide ester (Roche Diagnostics, Mannheim, Germany, Cat. No. 1 333 054) (3.8 mg/ml in DMSO) are added. After 30 min at room temperature, the sample is chromatographed on Superdex^{®} 200 (10 mM NaH₂PO₄/NaOH, pH 7.5, 30 mM NaCl). The fractions containing digoxigenylated IgG are collected. Monoclonal antibodies are labeled with digoxigenin according to the same procedure.

### Example 3

### Western Blotting for the detection of SPEE in human colorectal cancer tissue using polyclonal antibody as generated in Example 2

Tissue lysates from tumor samples and healthy control samples are prepared as described in Example 1, "Tissue preparation".

SDS-PAGE and Western-Blotting are carried out using reagents and equipment of Invitrogen, Karlsruhe, Germany. For each tissue sample tested, 10 µg of tissue lysate are diluted in reducing NuPAGE® (Invitrogen) SDS sample buffer and heated for 10 min at 95°C. Samples are run on 4-12% NuPAGE® gels (Tris-Glycine) in the MES running buffer system. The gel-separated protein mixture is blotted onto nitrocellulose membranes using the Invitrogen XCell II^{™} Blot Module (Invitrogen) and the NuPAGE^{®} transfer buffer system. The membranes are washed 3 times in PBS/0.05% Tween-20 and blocked with Roti®-Block blocking buffer (A151.1; Carl Roth GmbH, Karlsruhe, Germany) for 2 h. The primary antibody, polyclonal rabbit anti-SPEE serum (generation described in Example 2), is diluted 1:10,000 in Roti®-Block blocking buffer and incubated with the membrane for 1 h. The membranes are washed 6 times in PBS/0.05% Tween-20. The specifically bound primary rabbit antibody is labeled with a POD-conjugated polyclonal sheep anti-rabbit IgG antibody, diluted to 10 mU/ml in 0.5 x Roti®-Block blocking buffer. After incubation for 1 h, the membranes are washed 6 times in PBS/0.05% Tween-20. For detection of the bound POD-conjugated anti-rabbit antibody, the membrane is incubated with the Lumi-Light^{PLUS} Western Blotting Substrate (Order-No. 2015196, Roche Diagnostics GmbH, Mannheim, Germany) and exposed to an autoradiographic film.

Results of a typical experiment are shown in Figure 2.

### Example 4

### ELISA for the measurement of SPEE in human serum and plasma samples

For detection of SPEE in human serum or plasma, a sandwich ELISA is developed. For capture and detection of the antigen, aliquots of the anti-SPEE polyclonal antibody (see Example 2) are conjugated with biotin and digoxygenin, respectively.

Streptavidin-coated 96-well microtiter plates are incubated with 100 µl biotinylated anti-SPEE polyclonal antibody for 60 min at 10 µg/ml in 10 mM phosphate, pH 7.4, 1% BSA, 0.9% NaCl and 0.1% Tween-20. After incubation, plates are washed three times with 0.9% NaCl, 0.1% Tween-20. Wells are then incubated for 2 h with either a serial dilution of the recombinant protein (see Example 2) as standard antigen or with diluted plasma samples from patients. After binding of SPEE, plates are washed three times with 0.9% NaCl, 0.1% Tween-20. For specific detection of bound SPEE, wells are incubated with 100 µl of digoxygenylated anti-SPEE polyclonal antibody for 60 min at 10 µg/ml in 10 mM phosphate, pH 7.4, 1% BSA, 0.9% NaCl and 0.1% Tween-20. Thereafter, plates are washed three times to remove unbound antibody. In a next step, wells are incubated with 20 mU/ml anti-digoxigenin-POD conjugates (Roche Diagnostics GmbH, Mannheim, Germany, Catalog No. 1633716) for 60 min in 10 mM phosphate, pH 7.4, 1% BSA, 0.9% NaCl and 0.1% Tween-20. Plates are subsequently washed three times with the same buffer. For detection of antigen-antibody complexes, wells are incubated with 100 µl ABTS solution (Roche Diagnostics GmbH, Mannheim, Germany, Catalog No. 11685767) and OD is measured after 30-60 min at 405 nm with an ELISA reader.

### Example 5

### ROC analysis to assess clinical utility in terms of diagnostic accuracy

Accuracy is assessed by analyzing individual liquid samples obtained from well-characterized patient cohorts, i.e., 50 patients having undergone colonoscopy and found to be free of adenoma or CRC, 50 patients diagnosed and staged as T1-3, N0, M0 of CRC, and 50 patients diagnosed with progressed CRC, having at least tumor infiltration in at least one proximal lymph node or more severe forms of metastasis, respectively. CEA as measured by a commercially available assay (Roche Diagnostics, CEA-assay (Cat. No. 1 173 1629 for Elecsys^{®} Systems immunoassay analyzer) and SPEE measured as described above are quantified in a serum obtained from each of these individuals. ROC-analysis is performed according to Zweig, M. H., and Campbell, *supra.* Discriminatory power for differentiating patients in the group Tᵢₛ-3, N0, M0 from healthy individuals for the combination of SPEE with the established marker CEA is calculated by regularized discriminant analysis (Friedman, J. H., Regularized Discriminant Analysis, Journal of the American Statistical Association 84 (1989) 165-175).

Preliminary data indicate that SPEE may also be very helpful in the follow-up of patients after surgery.

### List of References

Ahlquist, D.A., Gastroenterol. Clin. North Am. 26 (1997) 41-55
Anderson, W.F., et al., J. Natl. Cancer Institute 94 (2002) 1126-1133
Bruck, C., et al., Methods Enzymol. 121 (1986) 587-695
Brünagel, G., et al., Cancer Research 62 (2002) 2437-2442
Carriquiry, L.A., and Pineyro, A., Dis. Colon Rectum 42 (1999) 921-929
Diamandis, et al., eds. (1996) Immunoassay, Academic Press, Boston.
Feuerstein, B.G., et al., Cancer Res. 45 (1985) 4950-4954
Friedman, J. H., Regularized Discriminant Analysis, Journal of the American Statistical Association 84 (1989) 165-175
Galfre, G., and Milstein, C., Methods Enzymol. 73 (1981) 3-46
Geenen, J.E., et al., Am. J. Dig. Dis. 20 (1975) 231-235
Goldenberg, D.M., et al., J. Natl. Cancer Inst. (Bethesda) 57 (1976) 11-22
Hannonen, P., et al., Biochim. Biophys. Acta 273 (1972) 84-90
Herrera, M.A., et al., Ann. Surg. 183 (1976) 5-9
Hibasami, H., et al., Anticancer Res. 7 (1987) 1213-1216
Hibasami, H., et al., FEBS Lett. 229 (1988) 243-246
Ito, H., et al., Cancer Lett. 15 (1982) 229-235
Janne, J., et al., Ann. Med. 23 (1991) 241-259
Kapyaho, K., et al., Biochem. J. 192 (1980) 59-63
Korpela, H., et al., Biochem. J. 196 (1981) 733-738
Linsalata, M., et al., Anticancer Res. 22 (2002) 2465-2469
Martell, R.E., et al., Int. J. Biol. Markers 13 (1998) 145-149
Moertel, C.G., et al., JAMA 270 (1993) 943-947
Myohanen, S., et al., DNA Cell Biol. 10 (1991) 467-474
Nishikawa, Y., et al., Biochem. J. 321 (1997) 537-543
Reynoso, G., et al., JAMA 220 (1972) 361-365
Scalabrino, G., and Ferioli, M.E., Adv. Cancer Res. 35 (1981) 151-268
Silvis, S.E., et al., JAMA 235 (1976) 928-930
Sobin, L.H., Wittekind, Ch. (eds), TNM Classification of Malignant Tumours, fifth edition, 1997
Studier, F.W., et al., Methods Enzymol. 185 (1990) 60-89
Sturgeon, C., Clinical Chemistry 48 (2002) 1151-1159
Tabor, C.W., and Tabor, H., Annu. Rev. Biochem. 53 (1984) 749-790
Tijssen P., *supra,* or Diamandis, et al., eds. (1996) Immunoassay, Academic Press, Boston
Tijssen, P., Practice and theory of enzyme immunoassays 11 (1990) the whole book, especially pages 43-78; Elsevier, Amsterdam
UICC (International Union Against Cancer), Sobin, L.H., Wittekind, Ch. (eds), TNM Classification of Malignant Tumours, fifth edition, 1997
Wahlfors, J., et al., DNA Cell Biol. 9 (1990) 103-110
Wahlfors, J., et al., DNA Cell Biol. 9 (1990) 543
Wanebo, H.J., et al., N. Engl. J. Med. 299 (1978) 448-451
Weiss, T.S., et al., Int. J. Colorectal Dis. 17 (2002) 381-387
Wingqvist, et al., Cytogenet. Cell Genet. 58 (1991) 1865
WO 01/96390
WO 02/078636
Zweig, M. H., and Campbell, G., Clin. Chem. 39 (1993) 561-577

## Claims

1. A method for the diagnosis of colorectal cancer comprising the steps of
a) providing a liquid sample obtained from an individual,
b) contacting said sample with a specific binding agent for protein spermidine synthase (SPEE) under conditions appropriate for formation of a complex between said binding agent and SPEE, and
c) correlating the amount of complex formed in (b) to the diagnosis of colorectal cancer.

2. The method according to claim 1, further **characterized in that** said sample is serum.

3. The method according to claim 1, further **characterized in that** said sample is plasma.

4. The method according to claim 1, further **characterized in that** said sample is whole blood.

5. Use of protein SPEE as a marker molecule in the diagnosis of colorectal cancer from a liquid sample obtained from an individual.

6. Use of protein SPEE as a marker molecule in the early diagnosis of colorectal cancer from a liquid sample obtained from an individual.

7. Use according to claim 6, wherein the early diagnosis is made with a sample derived from colorectal cancer patients in the adenoma stage.

8. Use according to claim 6, wherein the early diagnosis is made with a sample derived from colorectal cancer patients in stage Tᵢₛ -3; N0; M0

9. Use of protein SPEE as a marker molecule for colorectal cancer in combination with another marker molecule for colorectal cancer in the diagnosis of colorectal cancer from a liquid sample obtained from an individual.

## Patentansprüche

1. Verfahren zur Diagnose eines Kolorektalkarzinoms, das die folgenden Schritte enthält:
a) Bereitstellen einer einem Individuum entnommenen Flüssigkeitsprobe,
b) Inkontaktbringen der Probe mit einem spezifischen Bindungsmittel für das Protein Spermidinsynthase (SPEE) unter zur Ausbildung eines Komplexes zwischen dem Bindungsmittel und SPEE geeigneten Bedingungen und
c) Korrelieren der Menge an in (b) gebildetem Komplex mit der Kolorektalkarzinomdiagnose.

2. Verfahren nach Anspruch 1, ferner **dadurch gekennzeichnet, dass** es sich bei der Probe um Serum handelt.

3. Verfahren nach Anspruch 1, ferner **dadurch gekennzeichnet, dass** es sich bei der Probe um Plasma handelt.

4. Verfahren nach Anspruch 1, ferner **dadurch gekennzeichnet, dass** es sich bei der Probe um Vollblut handelt.

5. Verwendung des Proteins SPEE als Markermolekül bei der Diagnose eines Kolorektalkarzinoms aus einer einem Individuum entnommenen Flüssigkeitsprobe.

6. Verwendung des Proteins SPEE als Markermolekül bei der Frühdiagnose eines Kolorektalkarzinoms aus einer einem Individuum entnommenen Flüssigkeitsprobe.

7. Verwendung nach Anspruch 6, wobei die Frühdiagnose an einer aus Kolorektalkarzinompatienten im Adenomstadium stammenden Probe durchgeführt wird.

8. Verwendung nach Anspruch 6, wobei die Frühdiagnose an einer aus Kolorektalkarzinompatienten im Stadium Tᵢₛ -3; N0; M0 stammenden Probe durchgeführt wird.

9. Verwendung des Proteins SPEE als Markermolekül für Kolorektalkarzinome in der Kombination mit einem anderen Markermolekül für Kolorektalkarzinome bei der Diagnose von Kolorektalkarzinomen aus einer einem Individuum entnommenen Flüssigkeitsprobe.

## Revendications

1. Procédé pour le diagnostic d'un cancer colorectal comprenant les étapes de:
a) fourniture d'un échantillon liquide prélevé chez un sujet,
b) mise en contact dudit échantillon avec un agent de liaison spécifique de la protéine spermidine synthase (SPEE) dans des conditions appropriées pour la formation d'un complexe entre ledit agent de liaison et la SPEE, et
c) corrélation de la quantité de complexe formé en (b) au diagnostic d'un cancer colorectal.

2. Procédé selon la revendication 1, **caractérisé en outre en ce que** ledit échantillon est du sérum.

3. Procédé selon la revendication 1, **caractérisé en outre en ce que** ledit échantillon est du plasma.

4. Procédé selon la revendication 1, **caractérisé en outre en ce que** ledit échantillon est du sang total.

5. Utilisation de la protéine SPEE comme molécule marqueur dans le diagnostic d'un cancer colorectal à partir d'un échantillon liquide prélevé chez un sujet.

6. Utilisation de la protéine SPEE comme molécule marqueur dans le diagnostic précoce d'un cancer colorectal à partir d'un échantillon liquide prélevé chez un sujet.

7. Utilisation selon la revendication 6, dans laquelle le diagnostic précoce est réalisé avec un échantillon dérivé de patients atteints d'un cancer colorectal au stade d'adénome.

8. Utilisation selon la revendication 6, dans laquelle le diagnostic précoce est réalisé avec un échantillon dérivé de patients atteints d'un cancer colorectal au stade Tᵢₛ -3 ; N0 ; M0.

9. Utilisation de la protéine SPEE comme molécule marqueur du cancer colorectal en combinaison avec une autre molécule marqueur du cancer colorectal dans le diagnostic d'un cancer colorectal à partir d'un échantillon liquide prélevé chez un sujet.


### SEQUENCE LISTING

<110> Roche Diagnostics GmbH
   F. Hoffmann-La Roche AG
<120> Use of protein SPEE as a marker for colorectal cancer
<130> 21896
<150> EP 03017585.5
   <151> 2003-08-08
<160> 1
<170> PatentIn version 3.2
<210> 1
   <211> 300
   <212> PRT
   <213> Homo sapiens
<220>
   <221> MISC_FEATURE
   <223> spermidine synthase; SWISS-PROT: P19623
<400> 1
   Met Glu Pro Gly Pro Asp Gly Pro Ala Ala Ser Gly Pro Ala Ala Ile
   1               5                   10                  15
   Arg Glu Gly Trp Phe Arg Glu Thr Cys Ser Leu Trp Pro Gly Gln Ala
               20                  25                  30
   Leu Ser Leu Gln Val Glu Gln Leu Leu His His Arg Arg Ser Arg Tyr
           35                  40                  45
   Gln Asp Ile Leu Val Phe Arg Ser Lys Thr Tyr Gly Asn Val Leu Val
       50                  55                  60
   Leu Asp Gly Val Ile Gln Cys Thr Glu Arg Asp Glu Phe Ser Tyr Gln
   65                  70                  75                  80
   Glu Met Ile Ala Asn Leu Pro Leu Cys Ser His Pro Asn Pro Arg Lys
                   85                  90                  95
   Val Leu Ile Ile Gly Gly Gly Asp Gly Gly Val Leu Arg Glu Val Val
               100                 105                 110
   Lys His Pro Ser Val Glu Ser Val Val Gln Cys Glu Ile Asp Glu Asp
           115                 120                 125
   Val Ile Gln Val Ser Lys Lys Phe Leu Pro Gly Met Ala Ile Gly Tyr
       130                 135                 140
   Ser Ser Ser Lys Leu Thr Leu His Val Gly Asp Gly Phe Glu Phe Met
   145                 150                 155                 160
   Lys Gln Asn Gln Asp Ala Phe Asp Val Ile Ile Thr Asp Ser Ser Asp
                   165                 170                 175
   Pro Met Gly Pro Ala Glu Ser Leu Phe Lys Glu Ser Tyr Tyr Gln Leu
               180                 185                 190
   Met Lys Thr Ala Leu Lys Glu Asp Gly Val Leu Cys Cys Gln Gly Glu
           195                 200                 205
   Cys Gln Trp Leu His Leu Asp Leu Ile Lys Glu Met Arg Gln Phe Cys
       210                 215                 220
   Gln Ser Leu Phe Pro Val Val Ala Tyr Ala Tyr Cys Thr Ile Pro Thr
   225                 230                 235                 240
   Tyr Pro Ser Gly Gln Ile Gly Phe Met Leu Cys Ser Lys Asn Pro Ser
                   245                 250                 255
   Thr Asn Phe Gln Glu Pro Val Gln Pro Leu Thr Gln Gln Gln Val Ala
               260                 265                 270
   Gln Met Gln Leu Lys Tyr Tyr Asn Ser Asp Val His Arg Ala Ala Phe
           275                 280                 285
   Val Leu Pro Glu Phe Ala Arg Lys Ala Leu Asn Asp
       290                 295                 300